# EUROPÄISCHE PATENTSCHRIFT

(11) **EP 1 987 792 B1**
(45) Veröffentlichungstag und Bekanntmachung des Hinweises auf die Patenterteilung: **22.06.2011**
(21) Anmeldenummer: 07107464.5
(22) Anmeldetag: 03.05.2007
(51) Int. Cl.: A61B 17/62, A61B 17/64, A61B 17/80, A61B 17/86, A61B 19/00, F16B 2/00, F16B 9/00, F16B 35/00

(54) **Fixiervorrichtung, Kombination einer Fixiervorrichtung mit einem länglichen Element, Anordnung mit einer solchen Kombination sowie Osteosyntheseset**
Fixing device, combination of a fixing device with a long element, assembly with such a combination and osteosynthesis set
Dispositif de fixation, association d'un dispositif de fixation avec un élément longitudinal, dispositif doté d'une telle association tout comme kit d'ostéosynthèse

(43) Veröffentlichungstag der Anmeldung: 05.11.2008
(73) Patentinhaber: Medartis AG, 4057 Basel (CH)
(72) Erfinder: Schonhardt, Jürgen, 79618 Rheinfelden (DE); Tribelhorn, Thomas, 4434 Hölstein (CH); Zeuner, Hermann, 79111 Freiburg (DE); Norström, Joanna, 4053 Basel (CH)
(74) Vertreter: Hepp, Dieter

(56) Entgegenhaltungen:
- EP-A- 0 517 939
- WO-A-2004/086990
- WO-A-2005/013840
- WO-A2-2007/036807

## Beschreibung

Die Erfindung betrifft eine Fixiervorrichtung, eine Kombination einer solchen Fixiervorrichtung mit einem länglichen Element und eine entsprechende Anordnung mit einer Haltestruktur sowie ein Osteosyntheseset.

In der Knochenchirurgie werden verschiedene Arten von Fixiervorrichtungen verwendet. Bei verschiedenen Indikationen muss dabei ein längliches Element wie beispielsweise ein Kirschnerdraht an einer Haltestruktur wie beispielsweise einem internen oder externen Fixateur befestigt werden. Zur Befestigung der Drähte in der Haltestruktur sind verschiedene Anordnungen bekannt.

US 4 941 481 oder US 4 620 533 zeigen beispielsweise klammerartige Vorrichtungen, die in Art einer Klemmbacke über ein längliches Element geführt werden können und dieses dabei fixieren. Aus US 6 702 814 ist eine Klemmvorrichtung gezeigt, die für einen externen Fixateur verwendet werden kann und bei der zwei Klemmbacken einen Kanal zur Aufnahme eines länglichen Elements bilden. Eine der Klemmbacken ist zur Aufnahme des länglichen Elementes elastisch verformbar.

Aus US 5 393 191 ist ein externer Fixateur bekannt, bei dem ein längliches Element in eine geschlitzte Kugel eingeführt werden kann, welche zwischen zwei Klemmbacken einpressbar ist. Dadurch lässt sich die Orientierung des in der Kugel aufgenommenen länglichen Elements einstellen.

Aus EP 1 202 675 ist ein chirurgischer Führungskörper bekannt. Der Führungskörper dient zur Aufnahme von Fixationselementen und weist dazu eine Mehrzahl von windschief zueinander stehenden Öffnungen auf. Mittels eines Zwischenstücks lässt sich ein longitudinales Fixationselement wie beispielsweise ein Draht mittels Pressung, Klemmung oder Reibung fixieren.

Weitere Fixiervorrichtungen für längliche Elemente sind aus US 2 346 346, US 4 890 631, US 5 702 394, EP 1 408 859, WO 03/105704, EP 1 736 109, EP 1 570 796, US 4 621 627, EP 1 741 396, EP 1 306 057, US 4 135 505, US 4 127 119 oder DE 3439795, WO 03/065911 bekannt.

Alle diese bekannten Lösungen sind aber mit verschiedenen Nachteilen behaftet. Insbesondere bestehen viele der bekannten Lösungen aus einer Mehrzahl von einzelnen Elementen. Die Herstellung und Anwendung ist daher aufwändig und auch teuer. Ein weiterer Nachteil von bekannten Lösungen besteht darin, dass oft die zu fixierenden länglichen Elemente nur in eine Richtung oder nur mittels komplexen konstruktiven Lösungen multidirektional in verschiedene Richtungen befestigt werden können.

EP 1 202 675 zeigt zwar eine Lösung, bei der eine Position in verschiedenen Richtungen ohne komplizierte Kugelgelenke möglich ist. Verschiedene Richtungen sind aber auch hier nur in einer bestimmten, begrenzten Anzahl gemäss vorgeformten, windschiefen Öffnungen des Führungskörpers möglich. Eine Korrektur der Richtung ist nicht möglich.

WO 2005/013840 und WO 2007/036807 zeigen Klemmvorrichtungen, welche ein aussenliegendes Schraubengewinde bzw. eine sich in Umfangsrichtung radial wenigstens teilweise verringernden Klemmfläche aufweisen, das bzw. die zum Erzielen einer Kompression mit einem Innengewind bzw. einer Laufrille zusammenwirkt.

Die Veränderung der Winkellage einer solchen Klemmvorrichtung ist nicht oder nur dank zusätzlichen Bauteilen möglich.

Es ist daher eine Aufgabe der vorliegenden Erfindung, die Nachteile des Bekannten zu vermeiden, insbesondere also eine Fixiervorrichtung zum Fixieren von länglichen Elementen zu schaffen, welche einfach und kostengünstig herstellbar und auf eine einfache Art und Weise handhabbar ist. Trotzdem soll es die Fixiervorrichtung ermöglichen, die Richtung des zu fixierenden länglichen Elementes möglichst frei zu wählen und gegebenenfalls zu korrigieren. Eine weitere Aufgabe der vorliegenden Erfindung besteht darin, eine entsprechende Fixiervorrichtung zu schaffen, welche sich auf einfache Art und Weise re-positionieren lässt, beispielsweise zum Fixieren eines länglichen Elements unter einem anderen Winkel als ursprünglich vorgesehen.

Eine weitere Aufgabe der vorliegenden Erfindung besteht darin, eine solche Fixiervorrichtung in Kombination mit einem länglichen Element sowie eine Anordnung mit einer solchen Kombination und mit einer zusätzlichen Haltestruktur zu schaffen, welche die oben genannten Aufgaben ebenfalls lösen.

Ein weiterer Aspekt der Erfindung betrifft ausserdem ein Osteosyntheseset aus wenigstens zwei Implantatplatten und einer solchen Fixiervorrichtung. Vor allem bei der Versorgung von osteoporotischen Knochen besteht häufig das Problem, dass sich Implantatplatten mit Gewindeschrauben nicht ausreichend fest im Knochenmaterial verankern lassen. Es ist eine Aufgabe gemäss diesem Aspekt der Erfindung, ein Osteosyntheseset zu schaffen, welches sich zur Lösung der bekannten Probleme eignet, welches also insbesondere eine feste Fixierung der Implantatplatten auch ermöglicht, wenn auf Grund von nicht ausreichendem oder ungeeignetem Knochenmaterial eine herkömmliche Fixierung mit einer Schraube nicht möglich ist oder die Haltung der Schraube nicht genügend Stabilität bietet.

Erfindungsgemäss werden diese Aufgaben mit einer Fixiervorrichtung, einer Kombination einer solchen Fixiervorrichtung mit einem länglichen Element, einer Anordnung mit einer solchen Kombination sowie mit einem Osteosyntheseset mit den Merkmalen der unabhängigen Patentansprüche gelöst.

Die erfindungsgemässe Fixiervorrichtung dient zum Fixieren eines länglichen Elementes, insbesondere eines Drahtes, Stiftes, Rohrs oder eines Fadens, in einer Aufnahme einer Haltestruktur. Die Haltestruktur ist typischerweise eine Platte. Der Begriff Haltestruktur ist aber breit zu verstehen. Denkbar sind auch Anwendungen, bei denen die Haltestruktur in erster Linie die nachstehend beschriebenen Klemmkräfte aufbringt und nicht zum Halten der Fixiervorrichtung an sich. Die Fixiervorrichtung weist einen Grundkörper auf. Der Grundkörper ist mit einer Öffnung, insbesondere einer durchgehenden Bohrung zur Aufnahme des länglichen Elementes versehen. Die Öffnung kann sich in Längsrichtung erstrecken. Es ist aber auch denkbar, Öffnungen in Querrichtung (d.h. unter einem Winkel von mehr als 0° zur Längsachse) des Grundkörpers vorzusehen, beispielsweise für die Distraktion. Während die Öffnung zur Aufnahme des länglichen Elements typischerweise rund ausgebildet ist (v.a. wenn sie zur Aufnahme zylindrischen Gegenständen wie Drähten dient), sind auch andere Formen der Öffnung denkbar. Beispielsweise ist auch eine schlitzförmige Öffnung zur Aufnahme von flächigen länglichen Elementen denkbar. Anders gestaltete Öffnungen zur Aufnahme von profilierten länglichen Elementen sind ebenfalls denkbar.

Erfindungsgemäss ist der Grundkörper zumindest in einem äusseren Klemmbereich radial derart verformbar ausgebildet, dass die Dimension der Öffnung, insbesondere der Durchmesser der Bohrung reduzierbar ist. Unter äusserem Klemmbereich wird im Rahmen dieser Anmeldung derjenige Bereich verstanden, in welchem der Grundkörper durch Kontakt mit der Aufnahme in der Haltestruktur komprimiert ist. Der Grundkörper weist ausserdem eine Aussenkontur auf, die derart ausgebildet ist, dass beim Einsetzen der Fixiervorrichtung in die Öffnung der Haltestruktur der Klemmbereich radial so verformbar ist, dass das längliche Element in der Öffnung festklemmbar ist. Die Festklemmung kann dabei gesehen in der Richtung des länglichen Elementes axial auf gleicher Höhe erfolgen, auf der der Klemmbereich angeordnet ist. Es ist aber auch denkbar, eine Klemmung des länglichen Elementes axial beabstandet zum äusseren Klemmbereich des Grundkörpers vorzusehen. Das längliche Element kann vor dem Einsetzen in die Haltestruktur einfach in die Öffnung des Grundkörpers eingesetzt werden. Beim Einsetzen des Grundkörpers in die Aufnahme der Haltestruktur wird der Grundkörper auf Grund der gezielten Ausbildung seiner Aussenkontur radial komprimiert. Dadurch wird das längliche Element in der Öffnung des Grundkörpers festgeklemmt, so dass es in axialer Richtung nicht mehr verschoben werden kann. Die erfindungsgemässe Lösung ist besonders einfach, da neben dem länglichen Element und der Haltestruktur nur ein zusätzliches Bauteil, (die Fixiervorrichtung), notwendig ist, um das längliche Element zu fixieren.

Erfindungsgemäss ist die Fixiervorrichtung zumindest im Klemmbereich auf ihrer Aussenkontur mit wenigstens einer Klemmfläche versehen. Die Klemmfläche erweitert sich in Umfangsrichtung gesehen wenigstens teilweise radial. Ein Grundkörper mit einer solchen Struktur, jedoch ohne längliche Öffnung und ohne Schlitze, ist zum Erzeugen einer Verblockung einer Knochenschraube in einer Implantatplatte aus WO 04/086990 bekannt. Wenn der Schraubenkopf mit den Klemmflächen durch Rotation mit entsprechenden Keilvorsprüngen einer Aufnahme einer Haltestruktur in Kontakt gebracht wird, ergeben sich radiale Kräfte, welche zu einer Kompression des Grundkörpers führen. Es hat sich gezeigt, dass sich eine solche Verblockungsschraube besonders gut zum Fixieren von länglichen Elementen eignet, wenn sie mit einer länglichen Öffnung zur Aufnahme des Elementes sowie mit Mitteln zum Erleichtern der radialen Kompression wie beispielsweise Schlitzen versehen ist.

Die erfindungsgemässe Fixiereinrichtung weist ausserdem am Grundkörper auf dessen Aussenkontur in Längsrichtung gesehen im Bereich der Klemmfläche eine gerundete Aussenkontur, insbesondere eine wenigstens teilweise etwa sphärische, spiralförmige, parabolische, elliptische oder hyperbolische Kontur auf. Ganz allgemein ist eine Kontur bevorzugt, dank der sich die Richtung der Fixiervorrichtung in einer Aufnahme einer Haltestruktur in einen bestimmten Winkelbereich nahezu uneingeschränkt einstellen lässt. Dazu sind keine zusätzlichen Bauteile wie Kugelgelenke, Klemmbacken oder ähnliches notwendig. Die Aussenseite des Grundkörpers und/oder die Aufnahme der Haltestruktur kann ausserdem strukturiert werden, beispielsweise mit Rillen oder Rändeln versehen werden.

Gemäss einer ersten bevorzugten Ausführungsform der Erfindung ist die Fixiervorrichtung in ihrem Klemmbereich mit mindestens einem Schlitz versehen. Der Schlitz verläuft wenigstens teilweise in Richtung der Öffnung und endet wenigstens teilweise in der Öffnung. Der Schlitz kann bis zur Aussenwand der Fixiervorrichtung reichen. Der Schlitz muss nicht zwingend in einer Ebene verlaufen, er kann auch einen oder mehrere Knicke oder Krümmungen aufweisen. Auf Grund dieses wenigstens einen Schlitzes lässt sich der Grundkörper besonders einfach radial komprimieren. Ein Schlitz kann bereits ausreichend sein. Es kann aber auch eine beliebige grössere Anzahl von Schlitzen vorgesehen sein. Der oder die Schlitze sind beispielsweise in radialer Richtung angeordnet. Es ist aber auch denkbar, Schwächungen im Grundkörper durch tangentiale oder kurvenförmige Schlitze anzuordnen. Typischerweise verläuft der Schlitz genau in Richtung der Öffnung zur Aufnahme des länglichen Gegenstandes. Es ist aber auch denkbar, den Schlitz unter Winkeln bezogen auf die Achse der Fixiervorrichtung vorzusehen. Alternativ ist es aber auch denkbar, den Grundkörper aus einem elastischen Material auszubilden. In diesem Fall kann eine radiale Kompression auch ohne Schlitz erzielt werden. Ausserdem kann der Grundkörper zur Vereinfachung des Einsetzens des länglichen Elements auch aus mehreren, zueinander beweglichen Teilen ausgebildet sein. Denkbar sind zwei, mit einem Scharnier verbundene Teile. Diese Teile lassen sich zum Einsetzen des Elements aufklappen und wieder schliessen und dann miteinander verbinden. Die axiale Fixierung des länglichen Elementes erfolgt anschliessend wiederum in der vorstehend beschriebenen Art und Weise.

Es ist ausserdem auch denkbar, den Grundkörper aus mehreren Materialien und mit mehreren Komponenten auszubilden. Beispielsweise könnte elastisches Material für die Klemm-Zone und zähes Material für die Verblockungs- und Schraubendreher-Zone verwendet werden. Eine solche Fixiervorrichtung ist z.B. mit Mehrkomponenten-Spritzguss realisierbar.

Gemäss einer weiteren bevorzugten Ausführungsform der Erfindung ist die Fixiervorrichtung zumindest in ihrem Klemmbereich entsprechend als Schraubenkopf ausgebildet. Die Ausbildung als Schraubenkopf ermöglicht insbesondere ein einfaches Drehen der Fixiervorrichtung in Umfangsrichtung. Bei der vorstehend beschriebenen Ausbildung mit sich in Umfangsrichtung radial erweiternden Klemmflächen lässt sich die radiale Kompression auf besonders einfache Art und Weise durch Drehen mittels eines Schraubendrehers erzeugen, z.B. mit einem Kreuz-Schlitz, Torx, Innensechskant oder Variationen davon, wie beispielsweise der "HexaDrive" der Anmelderin.

Die Fixiervorrichtung kann an ihrem Grundkörper zusätzlich mit einem Schraubenschaft mit einem Schraubengewinde versehen sein. Es ist aber auch denkbar, die Fixiervorrichtung zusätzlich mit einem Schaft eines Pins zu versehen. Je nach Anwendung kann also eine Fixiervorrichtung, die ausschliesslich aus dem Grundkörper besteht oder eine Fixiervorrichtung, bei welcher der Grundkörper zusätzlich mit dem Schaft eines Pins oder einer Schraube versehen ist, vorteilhaft sein.

Gemäss einer weiter bevorzugten Ausführungsform der Erfindung kann die Fixiervorrichtung, wenn sie in ihrem Klemmbereich als Schraubenkopf ausgebildet ist, ausserdem oberhalb des Klemmbereichs mit einem Fortsatz versehen sein. Der Fortsatz ist als Aufnahme für einen Schraubendreher ausgebildet. Damit kann besonders einfach ein Drehmoment auf die Fixiervorrichtung aufgebracht werden.

Gemäss einem weiteren bevorzugten Ausführungsbeispiel der Erfindung erstreckt sich der wenigstens eine Schlitz in Längsrichtung der Fixiervorrichtung gesehen wenigstens über den Klemmbereich. Es ist aber auch denkbar, den Schlitz oder die Schlitze über einen längeren, axialen Bereich zu erstrecken, um die Verformbarkeit bzw. die Flexibilität des Grundkörpers weiter zu erhöhen. Maximal erstrecken sich die Schlitze über die ganze Länge der Fixiervorrichtung. Durch gezielte Wahl der Länge der Schlitze lässt sich die auf das längliche Element erzielbare Klemmwirkung einstellen.

Gemäss einer weiteren bevorzugten Ausführungsform der Erfindung kann die Innenfläche der Öffnung strukturiert sein. Auf diese Weise lässt sich durch zusätzlichen Formschluss eine auf Grund der radialen Kompression vergrösserte Rückhaltewirkung in axialer Richtung erzielen. Mit anderen Worten heisst dies, dass bei einer bestehenden Klemmkraft eine grössere Kraft auf dem länglichen Element in Achsrichtung notwendig wäre, um dies aus der Fixiervorrichtung auszureissen.

Entsprechend den vorstehenden Ausführungen besteht ein Aspekt der Erfindung auch darin, dass eine an sich bekannte Verblockungsschraube zusätzlich mit einer Längsbohrung versehen und im Bereich des Schraubenkopfes mit wenigstens einem Schlitz versehen wird. Besonders bevorzugt wird dabei eine Verblockungsschraube verwendet, wie sie in WO 2004/086990 gezeigt und beansprucht ist.

Ein weiterer Aspekt der Erfindung besteht daher in der Verwendung einer solchen Verblockungsschraube zum Fixieren eines länglichen Elementes in einer Haltestruktur. Durch die Verwendung einer multidirektionalen Verblockungsschraube lässt sich das längliche Element in Achsrichtung aber auch in einer bestimmten, in einem Winkelbereich frei wählbaren Winkellage fixieren.

Noch ein weiterer Aspekt der Erfindung betrifft die Kombination einer wie vorstehend beschriebenen Fixiervorrichtung mit einem länglichen Element. Dabei ist das längliche Element ausgewählt aus der Gruppe bestehend aus Drähten, Stäben, Stiften, Profilen, Federn, Hohlrohren oder einem Instrument zur Manipulation wie z.B. einem Skalpell oder Spatel (z.B. für Manipulationen am Gehirn). Wenn das längliche Element aus Draht, Stab oder Stift ausgebildet ist, sind beispielsweise Anwendungen zusammen mit einem Fixateur intern oder einem Fixateur extern möglich. Wenn das längliche Element als Profil oder Hohlrohr ausgebildet ist, sind beispielsweise auch Anwendungen als Endoskopierohr oder zur Fixierung einer Bohrerführung denkbar. Wenn das längliche Element als Halterung eines Skalpells ausgebildet ist, lässt sich ausserdem die erfindungsgemässe Fixiervorrichtung als Skalpellhalter zum Halten eines Skalpells einsetzen. Dabei kann der Schaft des Skalpells in die längliche Bohrung eingesetzt werden. Bei einer solchen Anwendung ist es aber auch denkbar, keine längliche Bohrung im Fixierelement vorzusehen, sondern direkt einen Teil der Klinge des Skalpells in am Fixierelement vorhandene Schlitze einzufügen.
Zum Erhöhen der axialen Klemmwirkung kann ausserdem auch das längliche Element eine strukturierte Oberfläche aufweisen.

Es ist ausserdem denkbar, je nach Anwendung das längliche Element mit einem Gewinde oder mit einem Rändel in Längs- oder Querrichtung oder auch mit einer Bohrerwendel zu versehen. Gemäss einer weiteren bevorzugten Ausführungsform kann das längliche Element mit einer Spitze versehen sein, die als Trokar oder Lanzette ausgebildet ist oder die mit einem selbstschneidenden oder selbstbohrenden Gewinde versehen ist.

Denkbar ist weiter ein Kabel/eine Schnur als längliches Element zur Übertragung von Zugkräften oder zur Verbindung von gerissenen Weichteilen wie Sehnen oder Bändern oder zur Fixierung von Knochenfragmenten.

Ein weiterer Aspekt der Erfindung betrifft eine Anordnung aus einer wie obenstehend beschriebener Kombination einer Fixiervorrichtung und eines länglichen Elementes zusammen mit einer Haltestruktur. Die Haltestruktur weist wenigstens eine Aufnahme für eine Fixiervorrichtung auf.

Die Aufnahme für die Fixiervorrichtung ist dabei besonders bevorzugt mit radial nach innen gerichteten Vorsprüngen oder Verjüngungen versehen. Diese Verjüngungen führen im Zusammenwirken mit den Klemmflächen an der Fixiervorrichtung zu einer radialen Kompression des Grundkörpers. Um auf möglichst einfache Weise ein multidirektionales Positionieren der Fixiervorrichtung und damit auch des darin gehaltenen länglichen Elementes zu ermöglichen, ist die Öffnung ausserdem bevorzugt mit einer Innenwand versehen, die in Längsrichtung wenigstens teilweise etwa sphärisch, spiralförmig, parabolisch, elliptisch oder hypobolisch ausgebildet ist. Insbesondere ist die Öffnung wie gemäss Offenbarung und Ansprüchen in WO 04/086990 ausgebildet.

Die Haltestruktur kann beispielsweise eine Haltestruktur zur Aufnahme eines Endoskopierohrs sein, welches mit der Fixiervorrichtung in der Haltestruktur gehalten wird. Dabei ist das längliche Element als Endoskopierohr ausgebildet.

Die Haltestruktur kann auch als temporäres, intraoperatives Stützelement ausgebildet sein, beispielsweise als Fixierring für ein Arthrodeseset.

Typische temporäre intraoperative Anwendungen sind Anwendungen, bei denen bestimmte Knochen zeitlich befristet fixiert werden sollen. Vor dem Ende des chirurgischen Eingriffs wird die entsprechende Stützstruktur entfernt. Indikationen sind beispielsweise Arthrodese oder Teilarthrodese des Handgelenks, die Neurochirurgie, Osteotomien, Korrekturen oder Frakturversorgungen sowie minimal-invasive Eingriffe, wie beispielsweise Transbuccal-Eingriffe oder Wirbelsäulenchirurgie. Als Fixateur extern wird eine Anwendung verstanden, bei der die stabilisierenden oder fixierenden Strukturen postoperativ ausserhalb des Körpers zur Stilllegung von Knochenbewegungen und damit zur Heilung von Frakturen verwendet werden. Es ist aber auch denkbar, gewisse Freiheitsgrade von Strukturen (z.B zwei durch ein Gelenk verbundene Knochen) zu fixieren und andere Freiheitsgrade der gleichen Struktur wiederum gezielt in ihrer Beweglichkeit zu belassen (dynamischer Fixateur Externe). Dies dient einer frühen postoperativen physiotherapeutischen Beübung zur Verhinderung von Verklebungen von Weichteilen und Versteifungen der betroffenen Gelenke. Typischerweise können dies Osteotomien, Korrekturen oder Frakturversorgungen sein. Denkbar ist ebenfalls die Anwendung bei einem Ringfixateur, wobei die Fixierung mehrerer Ringe untereinander ebenfalls über die erfindungsgemässe Fixiervorrichtung erfolgen kann. Denkbar ist überdies der Einsatz beispielsweise bei Oberarmfrakturen, wobei mit einem Gewindedraht die Gelenk-Kugel gefasst wird und über eine Mutter, die sich am Schraubenkopf abstützt die Position sehr fein eingestellt werden kann (pull-push-Instrument).

Die Haltestruktur kann ausserdem auch durch einen Operationsrahmen gebildet sein, der mit der Fixieranordnung an einem OP-Tisch befestigbar ist, beispielsweise in Anwendungen der Neurochirurgie zum Halten von Bohr- oder Führungshülsen oder von Spateln.

Bei der Anwendung als Fixateur intern verbleibt die Anordnung als Implantat zur Stilllegung von Knochenbewegungen und zur Heilung von Frakturen dauerhaft im Körper. Typische Anwendungen können Arthrodese oder Teilarthrodese, die Wirbelversteifung, die gelenksnahe Versorgung von Röhrenknochen, die Epiphyse, alle Osteotomien, Korrekturen oder Frakturversorgungen, die Drahtosteosynthese, ausgehend von einer Platte mit mindestens einem Loch oder die skelettale Verankerung in der Kieferorthopädie sein. Denkbar ist auch ein Drahtfixateur oder Kabelfixateur zum Erzeugen von Zugkräften bei Weichteilen.

Bei der Anwendung als Endoskop können durch die Bohrung in der Fixiervorrichtung Manipulationen im Körper (beispielsweise in der Neurochirurgie, in der Mund-, Kiefer-, oder Gesichtschirurgie, insbesondere transbuccal, oder bei der Chirurgie der Wirbelsäule oder des Beckens) vorgenommen werden.

In den drei letztgenannten Fällen ist das längliche Element typischerweise ein Draht bzw. ein Rohr/eine Hülse. Schliesslich kann die Haltestruktur auch als Halterung für ein Instrument wie ein Skalpell ausgebildet sein, wobei in diesem Fall das längliche Element der Klingenschaft des Skalpells oder ein Teil der Klinge ist.

Während vorliegend in erster Linie chirurgische Anwendungen beschrieben wurden, sind auch andere Anwendungen der erfindungsgemässen Fixiervorrichtung, der Kombination mit einem länglichen Element und der erfindungsgemässen Anordnung denkbar. Beispielsweise lassen sich solche Konstruktionen als Schnell-Spann-Systeme für K-Drähte/Bohrer bei Antriebs- oder Bohrmaschinen, als Spann-Mechanismus für Griffstücke z.B. in der Elektrochirurgie oder zur Knochen-Distraktion (z.B. von Gaumen, Alveolarkamm) einsetzen. Bei Anwendungen zur Distraktion kann das längliche Element z.B. zweiteilig ausgebildet sein, mit einem Teil mit einem Innen- und einem Teil mit einem Aussengewinde. Eine Fixiervorrichtung mit zwei quer angebohrten Grundkörpern dient zur Aufnahme je eines der Teile. Wenn die Grundkörper je in einem Knochenfragment verankert werden, lassen sich die Teile befestigen und zur Distraktion einsetzen.

Denkbar sind aber auch weitere Anwendungen z.B. im Baubereich wie z.B. im Regalbau, im Gerüstbau, in der Dübeltechnik (evtl. mit axialer Kompression) oder zur Abstützung.

Ein weiterer Aspekt der Erfindung betrifft ein Osteosyntheseset. Das Set besteht aus wenigstens zwei Implantatplatten beziehungsweise aus einer Implantatplatte mit wenigstens zwei Abschnitten. Die Implantatplatten bzw. die Abschnitte weisen jeweils wenigstens eine Aufnahme zur Aufnahme einer Verbindungsanordnung auf. Das Set weist ausserdem eine solche Verbindungsanordnung auf. Die Verbindungsanordnung ist in je eine Bohrung der Platten bzw. der Abschnitte einsetzbar. Dabei können die Implantatplatten mittels der Verbindungsanordnung der Implantatplatten miteinander verbunden und so stabilisiert werden. Die Platten werden daran gehindert, sich bezogen aufeinander entlang des länglichen Elements zu bewegen. Durch die feste Fixierung wird eine Verbindung einer oder mehrerer Platten untereinander erzeugt. An dieses Gerüst werden dann z.B. Fragmente reponiert und fixiert. Im Fall einer Platte mit zwei Abschnitten kann eine Verbindung von einem Abschnitt, z.B. einer Lasche einer Platte zu einem anderen Plattenabschnitt geschaffen werden. Dies kann z.B. sinnvoll sein, wenn die Verbindung zwischen den beiden Plattenabschnitten aus klinischen Gründen so ausgebildet sein muss, dass sie nur wenig oder keine Last übertragen kann oder sogar nach Herstellen der Verbindung aus klinischen Gründen entfernt wird.

Dank der Verbindungsanordnung lassen sich zwei oder mehr solcher Implantatplatten an mehreren Seiten eines Knochens befestigen/verbinden, selbst wenn der Knochen auf Grund von Grösse oder Struktur (innerer Beschaffenheit) zur Aufnahme einer Knochenschraube zum Fixieren der Implantatplatten nicht geeignet ist.

Denkbar ist es in diesem Zusammenhang auch, Implantatplatten mit laschenartigen, abgewinkelten Teilen zu versehen. Die Verbindungsanordnung kann dann in einer Aufnahme in dieser Lasche befestigt werden. Dies ist insbesondere vorteilhaft, wenn die vorstehend beschriebene Verbindung auf Grund der anatomischen Gegebenheiten mit flachen Platten nicht möglich ist, weil z.B. die Ebenen im Bereich der Aufnahme nicht in einem Winkel zueinander stehen, der kleiner ist als der Schwenkwinkel der Fixationselemente. Die abgewinkelten Teile bilden dann kleine Einheiten, die eine zur Verbindung brauchbare Position bieten.

Die Verbindungsanordnung weist eine Fixiervorrichtung in der eingangs beschriebenen Art und Weise auf. Damit lassen sich besonders einfach Spanndrähte in den Öffnungen der Implantatplatten befestigen. Für bestimmte Anwendungen wie beispielsweise craniomaxillofaciale Anwendungen ist es auch denkbar, den Winkel der Aufnahme bezogen auf die Platte schrägzustellen, also die Achse der Aufnahme nicht senkrecht zur Ebender Platte auszurichten. Damit kann eine Positionierung der Schrauben in einem weiteren Winkelbereichen erzielt werden, z.B. für eine um 30° schräg gestellte Aufnahme im Bereich von 15° (30°-15°) bis 45° (30°+15°). Insbesondere bei dünnen Platten kann in diesem Zusammenhang die Platte im Bereich der Aufnahme lokal verdickt werden. Damit wird Raum geschaffen zur schrägen Anordnung der Verblockungskontur in der Aufnahme.

Es ist aber auch denkbar, dass die Verbindungsanordnung ein längliches Element mit einem Gewinde an wenigstens einem Ende aufweist. Zusätzlich ist die Verbindungsanordnung in diesem Fall mit einem Verbindungselement wie z.B. einer Mutter versehen, in welche das Gewinde einschraubbar ist und mittels welcher das längliche Element in einer der Platten befestigbar ist.

Die Erfindung wird im Folgenden in Ausführungsbeispielen und an Hand der Zeichnungen näher erläutert. Es zeigen:
- Figur 1:: Seitenansicht einer erfindungsgemässen Fixiervorrichtung;
- Figur 2a:: Schnitt einer Fixiervorrichtung gemäss Figur 1 entlang der Ebene CC;
- Figur 2b:: zeigt einen Schnitt durch eine Ebene entlang eines Schlitzes der Fixiervorrichtung;
- Figur 3:: Draufsicht auf die Fixiervorrichtung gemäss Figur 1;
- Figur 4:: Untenansicht auf die Fixiervorrichtung gemäss Figur 1;
- Figur 5:: Schematische Darstellung einer als Schraube ausgebildeten erfindungsgemässen Fixiervorrichtung;
- Figur 6:: Perspektivische Darstellung einer Öffnung zur Aufnahme der erfindungsgemässen Fixiervorrichtung;
- Figur 7a:: Schematische Darstellung eines Ausführungsbeispiels einer nicht erfindungsgemässen Fixiervorrichtung;
- Figur 7b:: Schematische Darstellung eines alternativen Ausführungsbeispiels einer erfindungsgemässen Fixiervorrichtung
- Figur 8:: Schematische Darstellung eines Osteosynthesesets aus zwei Implantatplatten;
- Figuren 9a und b:: Alternative Ausführungsformen für ein Osteosyntheseset mit zwei Implantatplatten;
- Figur 10:: Schematische Darstellung der Anwendung einer erfindungsgemässen Fixiervorrichtung bei einem Fixateur extern
- Figur 11:: Schematische Darstellung der Anwendung von erfindungsgemässen Fixiervorrichtungen als temporäre intraoperative Fixierung;
- Figuren 12 bis 19:: Verschiedene Ausführungsformen von im Zusammenhang mit der vorliegenden Erfindung verwendbaren länglichen Elementen;

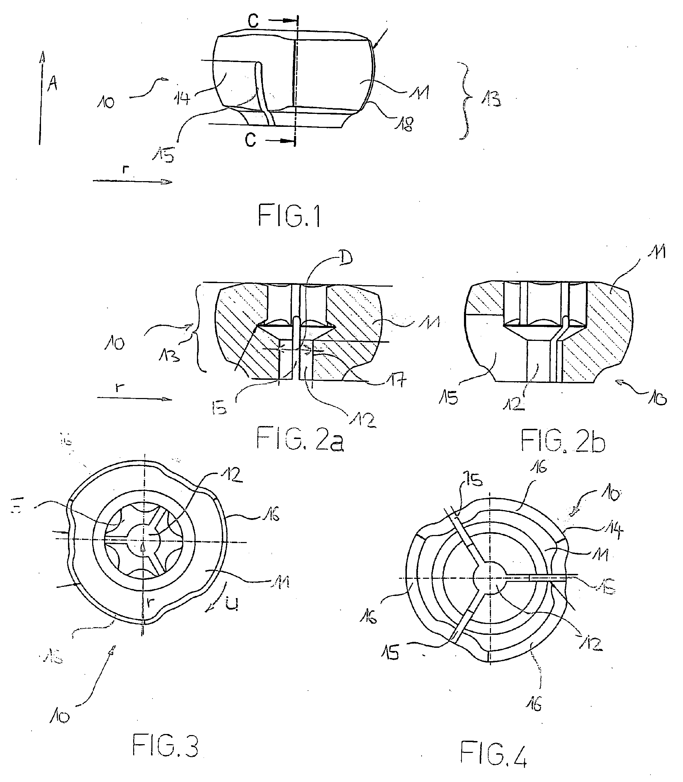

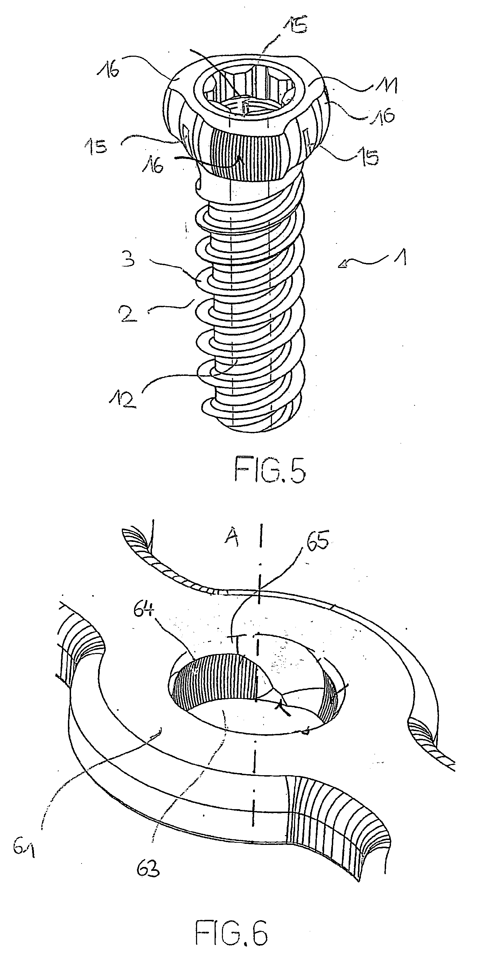

Figur 1 zeigt in Seitenansicht eine erfindungsgemässe Fixiervorrichtung 10. Die Fixiervorrichtung 10 weist einen Grundkörper 11 auf. Der Grundkörper 11 ist eine Aufnahme einer Haltestruktur, beispielsweise in die Aufnahme 63 in einer Implantatplatte 61 z.B. gemäss Figur 6 einsetzbar. Der Grundkörper weist einen Klemmbereich 13 auf. Im Klemmbereich 13 ist der Grundkörper 11 durch Kontakt mit der Innenwand 65 der Aufnahme 63 (s. Figur 6) komprimierbar. Der Grundkörper 11 weist ausserdem Schlitze 15 auf, die sich in Achsrichtung A erstrecken. Auf Grund der Schlitze 15 lässt sich der Grundkörper 11 in radialer Richtung r (d.h. in einer Ebene senkrecht zur Achsrichtung A) komprimieren. Der Grundkörper 11 weist eine Aussenkontur 14 auf. Die Aussenkontur 14 ist zumindest in einem Bereich 18 in Achsrichtung A gesehen gerundet ausgebildet. Durch die gerundete Aussenkontur 18 lässt sich der Grundkörper bzw. die Fixiervorrichtung in einer Vielzahl von unterschiedlichen Winkellagen in einer Aufnahme 63 einsetzen.

Der Grundkörper 11 ist ausserdem mit einer Bohrung 12 (s. Figur 2a und 2b) versehen. Die Bohrung 12 dient zur Aufnahme eines länglichen Elementes. Durch Kompression in radialer Richtung r lässt sich der Durchmesser D der Bohrung 12 reduzieren. Dadurch wird ein in die Bohrung 12 eingesetztes längliches Element wie typischerweise ein Draht festgeklemmt. Zur Erhöhung der Klemmwirkung kann ausserdem die Innenfläche 17 der Bohrung 12 mit einer Oberflächenstrukturierung versehen sein.

Figur 2b zeigt einen Schnitt durch den Grundkörper 11 in einer Ebene entlang des Schlitzes 15. Wie Figur 2b zeigt, erstreckt sich der Schlitz 15 vom einen Rand des Grundkörpers 11 bis zu der Bohrung 12.

Wie Figuren 3 und 4 zeigen, ist die Aussenkontur des Grundkörpers 10 in einer Ebene senkrecht zur Achsrichtung A mit drei Klemmflächen 16 versehen. Die Klemmflächen 16 weiten sich entgegen Umfangsrichtung U gesehen radial auf. Wenn der Grundkörper 11 in eine entsprechend ausgebildete Öffnung mit radialen Vorsprüngen 64 (s. Figur 6) eingesetzt und in Umfangsrichtung U verdreht wird, geraten die Klemmflächen 16 in Eingriff mit den Vorsprüngen 64. Daraus resultiert eine Kompression in radialer Richtung.

Zum Erzeugen einer Drehbewegung ist der Grundkörper 11 im Bereich der Bohrung 12 ausserdem mit einer Aufnahme 5 für ein entsprechendes Werkzeug, beispielsweise einen Schraubendreher versehen.

Figur 4 zeigt in Untenansicht die drei, jeweils 120° zueinander versetzt angeordneten, in Figur 4 genau radial verlaufenden Schlitze 15. Selbstverständlich sind andere Geometrien der Schlitze denkbar. Wie Figur 1 zeigt, erstrecken sich die Schlitze 15 etwa über zwei Drittel der Höhe des Grundkörpers 11. Selbstverständlich sind auch andere Dimensionierungen der Schlitzungen in Längsrichtung denkbar. Es ist auch denkbar, die Schlitze gekrümmt oder tangential auszubilden.

Die erfindungsgemässe Fixiervorrichtung ist bei medizinischen Anwendungen aus einem biokompatiblen Material gebildet. Typischerweise kann Titan verwendet werden. Andere wie z.B. rostfreie oder bioresorbierbare Materialien sind ebenfalls denkbar. Es können auch Legierungen mit Form-Gedächtniseigenschaft wie beispielsweise NiTiNol oder allgemein superelastische Materialien und beispielsweise auch Kunststoff verwendet werden.

Die Dimension der erfindungsgemässen Fixiervorrichtung richtet sich nach dem jeweiligen Anwendungsgebiet. Typischerweise sind für Anwendungen im Chirurgiebereich Fixiervorrichtungen mit einem Durchmesser von ca. 2 bis 7 mm, Bohrungen mit einem Durchmesser von ca. 0,75 bis 1,4 mm und Fixiervorrichtungen mit einer totalen Höhe von ca. 1 bis 5 mm denkbar. Die Schlitze können typischerweise eine Schlitzbreite von 0,2 bis 0,3 mm aufweisen und sich über eine Höhe von 0,3 bis 1,5 mm erstrecken. Variationen sind selbstverständlich je nach Anwendungsgebiet denkbar, so beispielsweise bei der Wirbelsäulenchirurgie, wo Endoskope mit Durchmessern von 10-50mm und Bohrungen mit Durchmessern von 5-40mm bekannt sind.

Figur 5 zeigt in dreidimensionaler Darstellung eine als Schraube 1 ausgebildete Fixiervorrichtung. An den Grundkörper 11 gemäss Figur 1 schliesst sich ein Schraubenschaft 2 an, der mit einem Schraubgewinde 3 versehen ist. Mit Ausnahme der Bohrung 12 und der Schlitze 15 ist die Schraube gemäss WO 04/086990 ausgebildet. Je nach Indikation kann der Schraubenschaft 2 mit dem Gewinde 3 vorteilhafte Eigenschaften haben. Es ist aber auch denkbar, den Schraubenschaft wegzulassen bzw. als Stift ohne Schraubgewinde auszubilden.

Figur 6 zeigt beispielhaft eine Aufnahme 63 für die Fixiervorrichtung 10. Die Aufnahme 63 weist eine Innenwand 65 auf. Die Innenwand 65 ist mit sich in radialer Richtung nach innen erstreckenden Vorsprüngen 64 versehen. In Figur 6 ist die Aufnahme 63 als Teil einer Implantatplatte 61 gezeigt. Die Aufnahme ist gemäss WO 04/086990 ausgebildet. Selbstverständlich können entsprechende Öffnungen in beliebigen Haltestrukturen vorgesehen werden, in welche mittels der Fixiervorrichtung längliche Elemente eingesetzt werden sollen.

Die in Figur 6 gezeigte Platte 61 mit der Öffnung 63 entspricht im Übrigen identisch der in WO 04/086990 gezeigten Platte. Insbesondere ist die Innenwandung 65 teilweise in Achsrichtung A sphärisch, spiralförmig, parabolisch, elliptisch oder hyperbolisch ausgebildet, so dass die Fixiervorrichtung mit ihrer gerundeten Aussenkontur 18 multidirektional in die Öffnung 63 einsetzbar und durch Drehen in Richtung U in der Öffnung 63 verklemmbar ist.

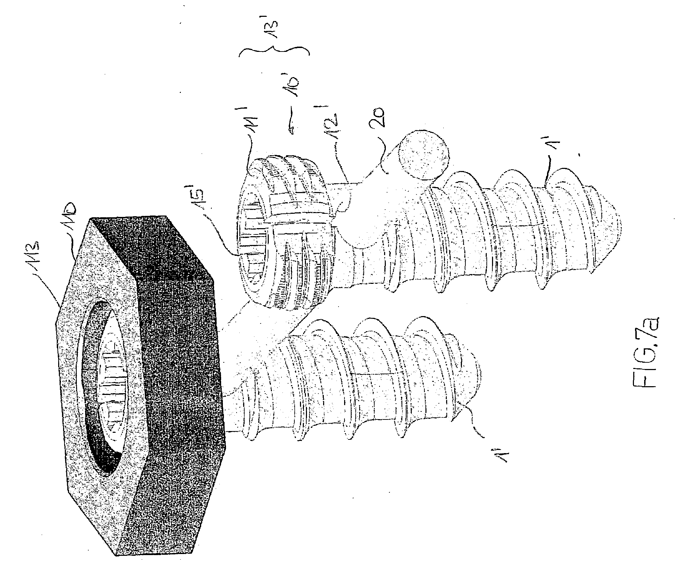

Figur 7a zeigt schematisch eine Ausführungsform einer nicht erfindungsgemässen Fixiervorrichtung. Die Fixiervorrichtung gemäss Figur 7a ist als Schraube 1' ausgebildet. Im Bereich des Schraubenkopfs 10' weist die Schraube 1' einen Grundkörper 11' auf. Der Grundkörper 11' ist mit einem sich in Achsrichtung der Schraube 1' erstreckenden Schlitz 15' versehen. Durch den Schlitz 15' wird ein Klemmbereich 13' gebildet. Der Grundkörper 11' ist ähnlich ausgebildet wie der in Figuren 1 bis 4 gezeigte Grundkörper. Durch Einsetzen des Grundkörpers 11' in eine Aufnahme 113 einer als Verriegelungselement 110 ausgebildeten Haltestruktur und durch Drehen lässt sich der Grundkörper 11' in einer Ebene senkrecht zur Achse der Schraube 1' komprimieren. Das heisst, dass die Breite des Schlitzes 15' dabei reduziert wird. Ein in eine quer zur Schraubenachse verlaufende Öffnung 12' eingesetzter Draht 20 wird dadurch in eine Richtung quer zur Schraubenachse festgeklemmt. Dabei ist es unerheblich, dass die Öffnung 12' in Achsrichtung gesehen unterhalb des Klemmbereichs 13' angeordnet ist. Die in Figur 7a gezeigte Anordnung kann beispielsweise zur Distraktion verwendet werden, wenn an Stelle eines Drahtes 20 ein zweiteiliges längliches Element mit Innengewinde bzw. Aussengewinde verwendet wird, welche ineinander eingefügt sind.

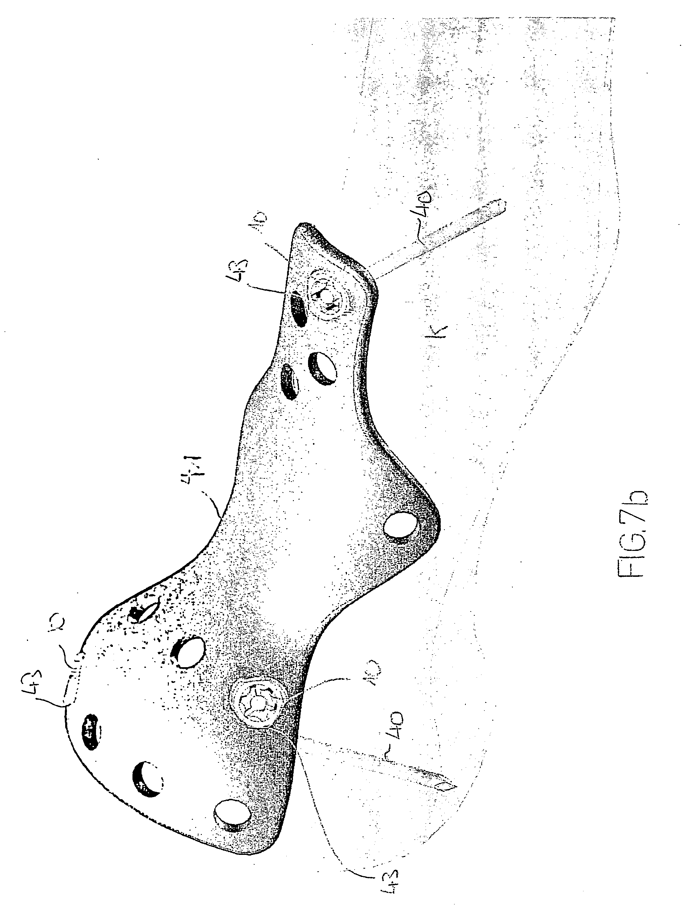

Figur 7b zeigt eine weitere alternative Ausführungsform. Eine Implantatplatte 41 ist als Fixateur intern für einen Radiuskopf K verwendet. Dazu werden in den Knochen K eingebrachte Drähte 40 in Öffnungen 43 an der Platte 41 mittels den vorstehend beschriebenen Fixiervorrichtungen 10 befestigt. Die Fixiervorrichtungen 10 ermöglichen eine axiale Fixierung der Drähte 40. Gleichzeitig können die Drähte unter verschiedenen Winkeln bezogen auf die Oberfläche der Platte eingesetzt und in ihrer Winkellage durch Verblockung positioniert werden. In Figur 7b ist eine Innenkontur zur Aufnahme eines Werkzeugs, typischerweise eines Schraubendrehers abgebildet. Mit diesem Werkzeug kann die Fixiervorrichtung 10 mit einem Drehmoment beaufschlagt, gedreht und dadurch verklemmt werden.

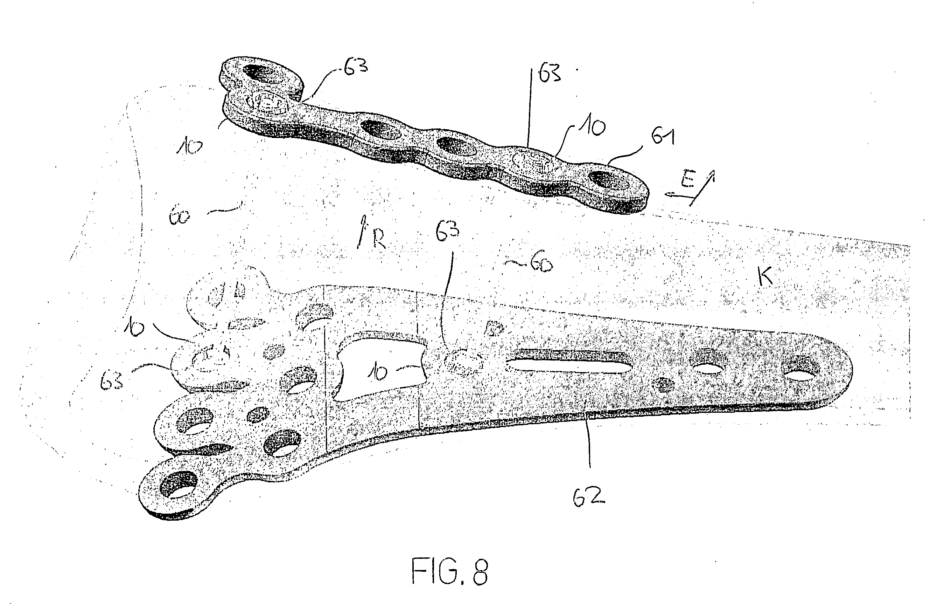

Figur 8 zeigt eine weitere mögliche Anwendung einer erfindungsgemässen Fixiervorrichtung. Zwei Implantatplatten 61, 62 werden auf zwei Seiten eines Knochens K positioniert. Zwei Drähte 60 werden durch den Knochen eingebracht und durch entsprechende Bohrungen 63 in den Implantatplatten 61, 62 geführt. Mittels der vorstehend beschriebenen Fixiervorrichtungen 10 werden die Drähte 60 in den Bohrungen 63 winkelstabil befestigt. Auf Grund der vorstehend beschriebenen freien Positionierbarkeit lässt sich die Richtung der Drähte 60 frei wählen. Mittels der Drähte 60 werden die beiden Implantate 61, 62 entlang der Drähte 60 im Bereich der Öffnungen 63 miteinander verbunden und dadurch stabilisiert. Auf diese Weise lässt sich eine Kraft aufbringen, selbst wenn das Knochenmaterial nicht in ausreichender Menge oder Qualität vorhanden ist, um eine herkömmliche Knochenschraube aufzunehmen.

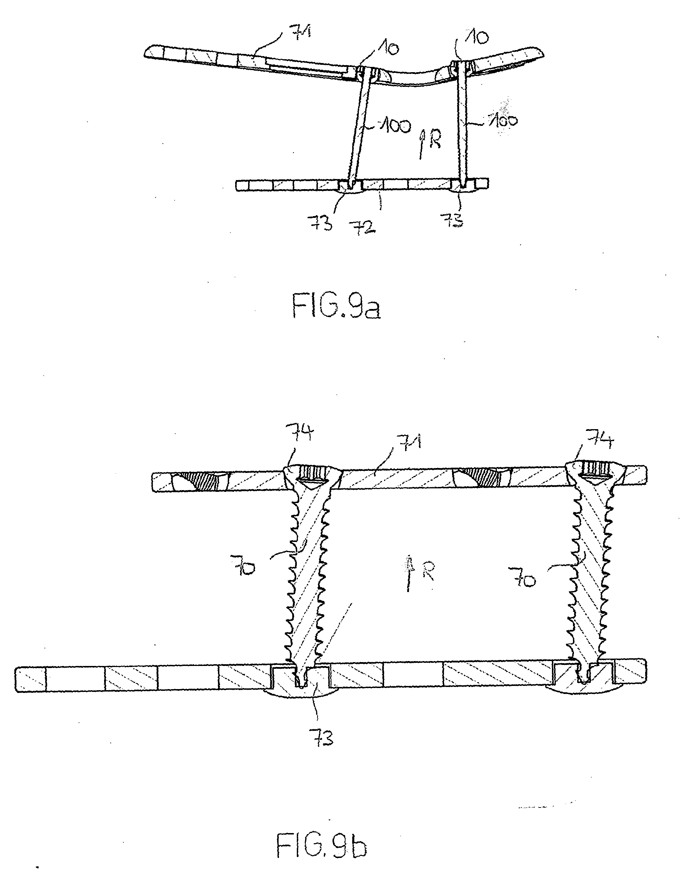

Das in Figur 8 gezeigte Osteosyntheseset aus den Platten 61, 62, den Drähten 60 und den Fixiervorrichtungen 10 ermöglicht die Anwendung von solchen Implantaten beispielsweise auch bei osteoporotischen Knochen. In Figuren 9a und 9b sind alternative Ausführungsformen von solchen Osteosynthesesets gezeigt.

Figur 9a zeigt ein alternatives Ausführungsbeispiel eines Osteosynthesesets. Gemäss Figur 9a werden zwei Implantatplatten 71, 72 mit Drähten 100 verbunden. Jeder Draht 100 ist ähnlich wie in Figur 8 gezeigt mittels einer Klemmvorrichtung 10 in einer Aufnahme der einen Implantatplatte 71 fixiert. Die Fixierung erfolgt im Wesentlichen wie im Zusammenhang mit Figur 8 erläutert. Im Bereich der zweiten Implantatplatte 72 weist der Draht 100 ein Gewinde auf. Eine Mutter 73, z.B. aus PEEK, ist auf das Gewinde geschraubt und fixiert so den Draht 100 im Bereich der zweiten Implantatplatte 72. Auch mit einer derartigen Anordnung lässt sich die Stabilisierung in Richtung R erzeugen, wobei R nicht senkrecht zur Plattenebene ausgerichtet sein muss.

In Figur 9b sind zwei Implantatplatten 71, 72 mit Schrauben 70 miteinander verbunden. Eine Mutter 73 oder ein Plättchen mit einer Öffnung ist im Bereich der einen Platte 72 mit dem Ende der Schraube 70 verbunden. Die Schrauben 70 stützten sich mit ihren Schraubenköpfen 74 auf der anderen Platte 71 ab. Auch mit dieser Stabilisierung lässt sich eine Verbindung der Implantatplatten 71, 72 in Richtung R erzielen, wobei R nicht senkrecht zur Plattenebene ausgerichtet sein muss.

Als Mutter wird hier insbesondere jede Art von Widerlager für den Draht verstanden. Die Mutter muss nicht zwingend parallel zu der Plattenebene angeordnet sein. Auch die Mutter kann einen gewissen Schwenkbereich haben. Auch die Mutter kann verblockbar sein. Schliesslich muss die Mutter nicht zwingend eine Öffnung aufweisen. Eine selbstbohrende Drahtspitze kann sich auch ein Loch bohren. Die Mutter kann ausserdem auch in der Platte vormontiert sein.

Zum Verbinden der Platten gemäss Figuren 7, 7b, 8, 9a und 9b und zur Stabilisierung der Fragmentpositionen muss ein Draht gesetzt werden. Dafür wird ein Zielgerät verwendet, das den Draht in das Start- und Ziel-Loch einfädelt. Die Verbindungslinie dieser beiden Punkte entspricht nach dem Einbringen des Drahtes der Drahtachse. Die Klemmelemente werden anschliessend aufgefädelt und durch Drehung in Umfangsrichtung U in der Öffnung blockiert. Eine Verschwenkung der Fixiervorrichtung 10 ist auf Grund der abgerundeten Aussenkontur in Achsrichtung gesehen möglich, jedoch durch die Geometrie der Implantatplatten 61, 62 auf einen gewissen Winkelbereich, typischerweise maximal +/-**15°** begrenzt.

Durch diese Schwenkbarkeit kann man die Platten exakt nach anatomischen Gegebenheiten platzieren ohne dabei die anschliessende Verbindung der Platten berücksichtigen zu müssen. Bei nichtmultidirektionalen, nicht-schwenkbaren Systemen müssten Kompromisse bei der Anpassung an anatomische Gegebenheiten eingegangen werden.

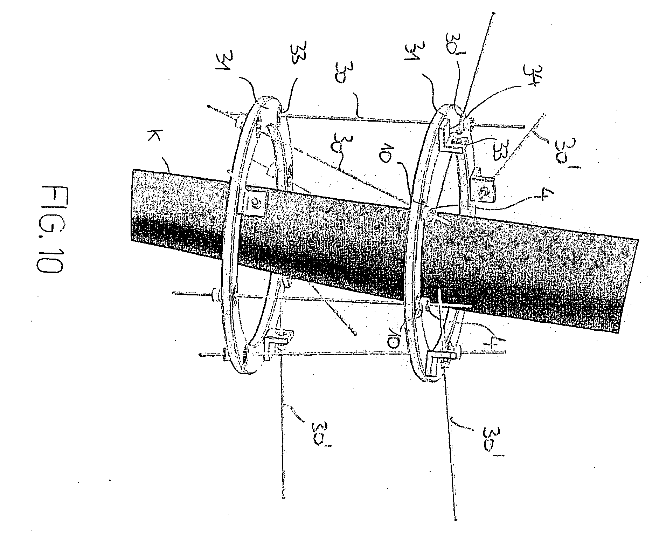

Figur 10 zeigt schematisch die Anwendung von erfindungsgemässen Fixiervorrichtungen bei einem Fixateur extern. Der Fixateur extern, typischerweise für einen Humerus weist zwei Ringe 31 auf. Die Ringe 31 sollen miteinander durch Drähte 30 stabilisiert werden. Gleichzeitig soll das Gebilde aus den beiden Ringen 31 und den etwa in Richtung des Knochens K verlaufenden Drähten 30 mit quer zum Knochen verlaufenden Drähten 30' an diesem fixiert werden. Zur Befestigung der Drähte 30, 30' in den Ringen sind in Öffnungen 33 der Ringe bzw. in Öffnungen 33 von auf den Ringen aufgebrachten Zusatzelementen 34 Fixiervorrichtungen 10 angebracht. Die Fixiervorrichtungen 10 und die Öffnungen 33 sind wie vorstehend beschrieben ausgebildet. Zusätzlich können die Fixiervorrichtungen 10 mit einem Fortsatz 4 versehen sein, welcher zur Aufnahme eines Werkzeugs oder zur (manuellen) Manipulation dient.

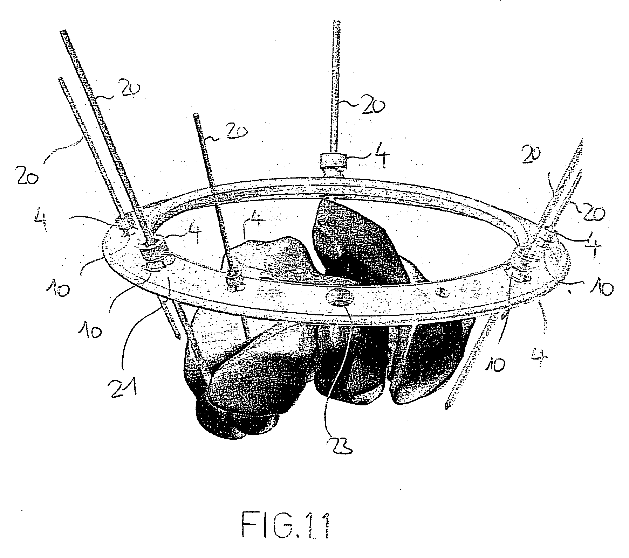

Figur 11 zeigt schematisch eine Anwendung der erfindungsgemässen Fixiervorrichtung 10 zur temporären, intraoperativen Fixierung des Mittelhandknochens. Ein Fixierring 21 liegt auf der Hand auf oder schwebt darüber und ist am OP Tisch befestigt, z.B. mit einer wie vorstehend beschriebenen Fixiervorrichtung. Fixierdrähte 20 dienen zur temporären Fixierung. Die Drähte 20 werden durch die Bohrung 12 (siehe z.B. Fig. 2a und 2b) der Fixiervorrichtung 10 geführt. Die Fixiervorrichtungen 10 sind in entsprechende Öffnungen 23 auf den Fixierring 21 eingedreht. Durch Drehung der Fixiervorrichtung in der Umfangsrichtung U (s. auch Figur 3) lassen sich die Fixiervorrichtungen 10 in den Öffnungen 23 fixieren und radial komprimieren. Dadurch wird der Draht 20 in Achsrichtung in der Fixiervorrichtung 10 fixiert der Fixierring wird auch gegen Verkippen fixiert. Die Öffnungen 23 sind ähnlich ausgebildet wie die im Zusammenhang mit der Implantatplatte in Figur 6 dargestellte Öffnung. Je nach Anwendung können unterschiedliche Grössen von Fixiervorrichtungen 10 eingesetzt werden.

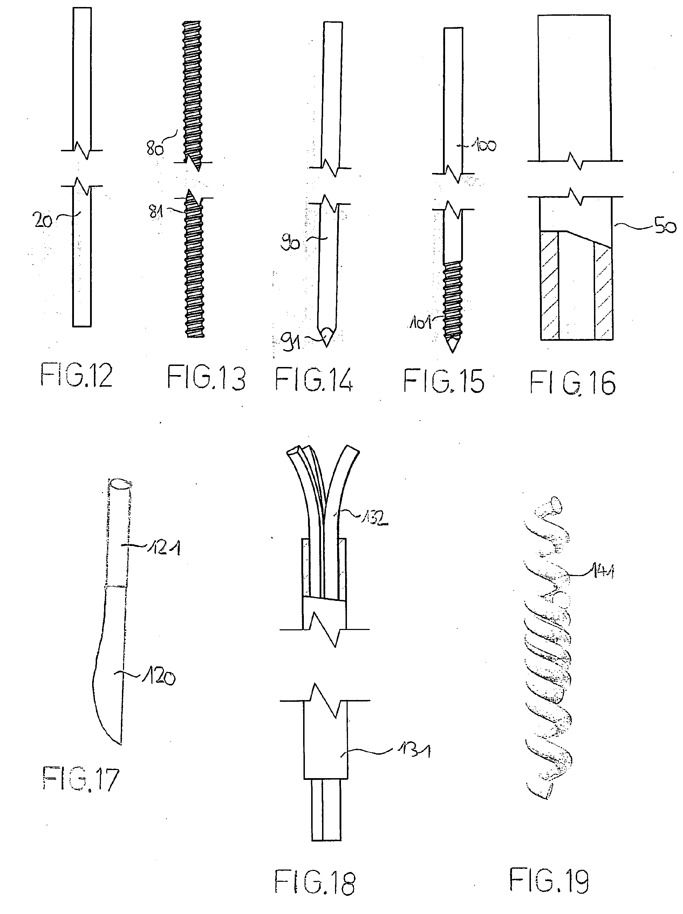

Die Figuren 12 bis 19 zeigen verschiedene Ausführungsformen von länglichen Elementen, welche in die erfindungsgemässe Fixiervorrichtung 10 einsetzbar sind.

Figur 12 zeigt schematisch einen einfachen Draht 20 ohne bestimmte Konturierung der Oberfläche.

Figur 13 zeigt einen Draht 80, der auf seiner ganzen Länge mit einem Gewinde 81 versehen ist. Typische Anwendungen sind beispielsweise K-Drähte mit kurzem (ca.20mm) Gewinde oder mit einem Gewinde auf der ganzen Länge. Das Gewinde an der Spitze kann z.B. dazu dienen, ein Knochenfragment gegen axiales Verschieben auf dem K-Draht zu sichern. Das Gewinde am gesamten Schaft stellt ausserdem eine kostengünstige Strukturierung quer zur Längsachse dar, die ausserdem zur Verstärkung der Haltekraft dienen kann. Statt einer Strukturierung der Oberfläche im Sinne eines Gewindes 81 sind auch in Längsrichtung oder in Querrichtung verlaufende Rändel denkbar.

Figur 14 zeigt ein drahtähnliches Element 90, welches an seinem Ende mit einem Trokar-Schliff 91 versehen ist.

Figur 15 zeigt ein drahtähnliches Element 100, welches nur an seiner Spitze mit einem Gewinde 101 versehen ist.

Figur 16 zeigt ein Endoskopierohr 50. Ein rohrförmig ausgebildetes längliches Element wie in Figur 16 gezeigt, kann auch im Zusammenhang mit einem Lanzettenschliff verwendet werden.

Figur 17 zeigt ein Skalpell 120, das mit seinem Schaft 121 in einer erfindungsgemässen Fixiervorrichtung befestigt werden kann.

Figur 18 zeigt ein rohrförmiges Element 131, das zur Aufnahme eines Drahtbündels 132 dient. Im osteoporotischen Knochen des Oberarms besteht die Gefahr, dass die Gelenkfläche kollabiert. Wenn das Element 131 in einer am Schaft eines Knochens verankerten Platte fixiert wird, können die Enden des Drahtbündels den osteoporotischen Knochen im Bereich der Gelenkfläche von innen abstützen. Das Rohrförmige Element übernimmt dann die Funktion eines länglichen Elementes, welches in einer wie vorstehend beschriebenen Fixiervorrichtung 10 (siehe z.B. Fig. 1 und 2) fixiert wird. Denkbar ist es auch, das Rohr mit einer geeigneten Aussenkontur (nicht gezeigt) zu versehen, so dass das Rohr die Aufgabe einer erfindungsgemässen Fixiervorrichtung zum Halten des Drahtbündels erfüllt.

Aber auch ein K-Draht mit einer grossen Stirnfläche mit einem Element, das sich aufklappt oder ein geschlitzter Draht, kann zu diesem Zweck eingesetzt werden.

Figur 19 zeigt ein federartiges Element 141. Das federartige Element 141 besteht aus zwei ineinander gesteckte Spiralfedern. Als Federelement sind auch Blattfedern, Spiralfedern denkbar, aber auch eine Feder aus zwei ineinander geschobenen Spiralfedern.

Je nach Anwendung bestehen die länglichen Elemente aus geeigneten Materialien. Typischerweise können bei implantierbaren Elementen Titan verwendet werden. Ebenfalls denkbar sind rostfreie Materialien, Form-Gedächtnis-Legierungen, Elemente aus superelastischen Werkstoffen oder je nach Anwendung auch Kunststoff.

## Patentansprüche

1. Fixiervorrichtung (10, 10'), zum Fixieren eines länglichen Elementes (20, 30, 30', 40, 50, 60, 80, 90, 100, 120, 131, 141), insbesondere eines Drahtes, Stiftes oder Rohrs, in einer Aufnahme (23, 33, 43, 63, 113) einer Haltestruktur (21, 31, 41, 61, 62, 71, 110), insbesondere einer Platte,
mit einem Grundkörper (11, 11'), der eine Öffnung (12, 12') (A), insbesondere eine durchgehende Bohrung, zur Aufnahme des länglichen Elementes aufweist, wobei
der Grundkörper (11, 11') zumindest in einem Klemmbereich (13, 13') bezogen auf das längliche Element radial derart verformbar ist, dass die Dimension (D) der Öffnung, insbesondere der Durchmesser der Bohrung (12) reduzierbar ist,
wobei der Grundkörper (11, 11') eine Aussenkontur (14) aufweist, die derart ausgebildet ist, dass beim Einsetzen der Fixiervorrichtung (10, 10') in die Aufnahme (23, 33, 43, 63, 113) der Klemmbereich (13) so verformbar ist, dass das längliche Element (20, 30, 30', 40, 50, 60, 80, 90, 100, 120, 131, 141) in der Öffnung, insbesondere Bohrung (12), festklemmbar ist,
wobei die Fixiervorrichtung (10, 10') im Klemmbereich (13) auf der Aussenkontur (14) mit wenigstens einer, sich in Umfangsrichtung (U) radial wenigstens teilweise verringernden Klemmfläche (16) versehen ist, die durch Rotation des Grundkörpers mit entsprechenden Keilvorsprüngen einer Aufnahme (23, 33, 43, 63, 113) einer Haltestruktur (21, 31, 41, 61, 62, 71, 110) in Kontakt bringbar ist, sodass sich radiale Kräfte ergeben, welche zu einer radialen Kompression des Grundkörpers führen, **dadurch gekennzeichnet dass**
die Fixiervorrichtung (10, 10') einen Grundkörper (11, 11') aufweist, der wenigstens teilweise in Längsrichtung (A) gesehen eine gerundete Aussenkontur (18) aufweist, so dass sich die Richtung der Fixiervorrichtung (10, 10') in der Aufnahme (23, 33, 43, 63, 113) der Haltestruktur (21, 31, 41, 61, 62, 71, 110) in einem bestimmten winkelbereich einstellen lässt, wobei die gerundete Aussenkontur (18) insbesondere annährend sphärisch, spiralförmig, parabolisch, elliptisch oder hyperbolisch ausgebildet ist.

2. Fixiervorrichtung nach Anspruch 1, **dadurch gekennzeichnet, dass** die Fixiervorrichtung im Klemmbereich (13) mit wenigstens einem Schlitz (15) versehen ist, der wenigstens teilweise in Richtung der Öffnung (12) verläuft und wenigstens teilweise in dieser endet.

3. Fixiervorrichtung nach einem der Ansprüche 1 bis 2, **dadurch gekennzeichnet, dass** die Fixiervorrichtung (10, 10') im Klemmbereich (13) als Schraubenkopf ausgebildet ist.

4. Fixiervorrichtung nach Anspruch 3, **dadurch gekennzeichnet, dass** die Fixiervorrichtung (10, 10') als Schraube (1) mit einem Schraubenschaft (2) mit einem Schraubengewinde (3) oder als Pin mit einem Schaft ohne Schraubengewinde ausgebildet ist.

5. Fixiervorrichtung nach einem der Ansprüche 3 oder 4, **dadurch gekennzeichnet, dass** der Schraubenkopf (2) insbesondere oberhalb des Klemmbereichs (13) mit einem Fortsatz (4) zum Beaufschlagen der Fixiervorrichtung (10, 10') mit einem Drehmoment versehen ist.

6. Fixiervorrichtung nach einem der Ansprüche 2 bis 5, **dadurch gekennzeichnet, dass** der Schlitz (15) wenigstens im Klemmbereich (13) und bis maximal entlang der ganzen Länge des Grundkörpers (11, 11') angeordnet ist.

7. Fixiervorrichtung nach einem der Ansprüche 1 bis 6, **dadurch gekennzeichnet, dass** die Öffnung (12) eine Innenfläche (17) aufweist, welche wenigstens teilweise strukturiert ist.

8. Kombination einer Fixiervorrichtung nach einem der Ansprüche 1 bis 7 und einem länglichen Element (20, 30, 30', 40, 50, 60, 80, 90, 100, 120, 131, 141), wobei das längliche Element ausgewählt ist aus der Gruppe, bestehend aus Draht, Stab, Stift, Profil, Feder, Hohlrohr, Skalpell.

9. Kombination nach Anspruch 8, **dadurch gekennzeichnet, dass** das längliche Element (80, 100) wenigstens teilweise eine strukturierte oberfläche aufweist.

10. Kombination nach einem dieser Ansprüche 8 oder 9, **dadurch gekennzeichnet, dass** das längliche Element wenigstens teilweise mit einem Gewinde, einer Bohrerwendel und/oder einem Rändel (80, 100) versehen ist.

11. Kombination nach einem der Ansprüche 8 oder 9, **dadurch gekennzeichnet, dass** das längliche Element (90, 100) eine Spitze (91) aufweist, die als Lanzette oder Trokar ausgebildet ist, oder die mit einem selbstschneidenden oder selbstbohrenden Gewinde (101) versehen ist.

12. Anordnung mit einer Kombination nach einem der Ansprüche 8 bis 11 sowie mit einer Haltestruktur (21, 31, 41,, 61, 62, 71, 110) mit wenigstens einer Aufnahme (23, 33, 43, 63, 113) zur Aufnahme der Fixiervorrichtung (10, 10').

13. Anordnung nach Anspruch 12, **dadurch gekennzeichnet**, die Aufnahme und die Fixiervorrichtung derart ausgebildet sind, dass das längliche Element mit der Fixiervorrichtung in einer Mehrzahl von unterschiedlichen Winkelpositionen bezogen auf die Haltstruktur befestigbar ist.

14. Anordnung nach Anspruch 12 oder 13, **dadurch gekennzeichnet, dass** die wenigstens eine Aufnahme (23, 33, 43, 63, 113) an ihrer Innenwandung (65) mit wenigstens einer sich radial nach innen erstreckenden Verjüngung (64) versehen ist.

15. Anordnung nach einem der Ansprüche 12 bis 14, **dadurch gekennzeichnet, dass** die Innenwandung (65) in Längsrichtung (A) gesehen mit einer gerundeten Innenkontur versehen ist, insbesondere zumindest annähernd sphärisch, spiralförmig, parabolisch, elliptisch oder hyperbolisch ausgebildet ist.

16. Anordnung nach einem der Ansprüche 12 bis 15, **dadurch gekennzeichnet, dass** die Haltestruktur ausgewählt ist aus der Gruppe bestehend aus
- einer Haltestruktur (51) zur Aufnahme eines Endoskopierohres (50), wobei das längliche Element als Rohr oder Trokar ausgebildet ist,
- einem temporären, intraoperativen Stützelement (21), insbesondere ein Fixierring für ein Arthrodeseset,
- einem Operationsrahmen, der am OP-Tisch fixierbar ist
- einem Fixateur intern (41), Drahtfixateur, Kabelfixateur oder intramedullären Marknagel
- einem Fixateur extern (31)
- einer Skalpellhalterung, wobei das längliche Element als Skalpellklinge (120) oder als Skalpellschaft (121) ausgebildet ist.

17. Osteosyntheseset, bestehend aus mindestens zwei Implantatplatten (61, 62; 71, 72) oder aus einer Implantatplatte mit wenigstens zwei Abschnitten,
welche mit jeweils wenigstens einer Aufnahme (63) zur Aufnahme einer Verbindungsanordnung (10, 60; 70, 73; 10, ,73, 100) versehen sind
und mit wenigstens einer Verbindungsanordnung (10, 60; 70, 73; 10, 73, 100), welche in je eine Aufnahme (63) der Implantatplatten (61, 62; 71, 72) oder der Abschnitte einsetzbar ist,
wobei die Implantatplatten (61, 62; 71, 72) oder deren Abschnitte mittels der Verbindungsanordnung (10, 60; 70, 73; 10, 73, 100) der Implantatplatten miteinander verbindbar und stabilisierbar sind, wobei
die Verbindungsanordnung wenigstens eine Fixiervorrichtung (10, 10') nach einem der Ansprüche 1 bis 7 sowie wenigstens ein Längliches Element, insbesondere einen Verbindungsdraht (60, 70, 100), aufweist.

18. Osteosyntheseset nach Anspruch 17, **dadurch gekennzeichnet, dass** das längliche Element (60, 70, 100) ein Gewinde an wenigstens einem Ende und ein Verbindungselement, insbesondere eine Mutter (73), aufweist, in welche das Gewinde einschraubbar ist und mittels welcher das längliche Element (60, 70, 100) in einer der Implantatplatten (72) befestigbar ist.

## Claims

1. Fixing device (10, 10'), for fixing an elongated element (20, 30, 30', 40, 50, 60, 80, 90, 100, 120, 131, 141), in particular a wire, pin or tube, in a receptacle (23, 33, 43, 63, 113) of a retaining structure (21, 31, 41, 61, 62, 71, 110), in particular a plate,
with a main body (11, 11'), which has an opening (12, 12'), in particular a through-bore, for receiving the elongated element,
wherein, at least in a clamping region (13, 13'), the main body (11, 11') is radially deformable in relation to the elongated element in such a way that the dimension (D) of the opening, in particular the diameter of the bore (12), can be reduced,
wherein the main body (11, 11') has an outer contour (14) which is formed in such a way that, when the fixing device (10, 10') is inserted into the receptacle (23, 33, 43, 63, 113), the clamping region (13) can be deformed such that the elongated element (20, 30, 30', 40, 50, 60, 80, 90, 100, 120, 131, 141) can be securely clamped in the opening, in particular the bore (12),
wherein, in the clamping region (13), the fixing device (10, 10') is provided on the outer contour (14) with at least one clamping area (16), which at least partially reduces radially in the circumferential direction (U) and, by rotation of the main body, can be brought into contact with corresponding wedge projections of a receptacle (23, 33, 43, 63, 113) of a retaining structure (21, 31, 41, 61, 62, 71, 110), so that radial forces that lead to a radial compression of the main body are obtained, **characterized in that**
the fixing device (10, 10') has a main body (11, 11'), which has, seen in the longitudinal direction (A), at least partially a rounded outer contour (18), so that the direction of the fixing device (10, 10') in the receptacle (23, 33, 43, 63, 113) of the retaining structure (21, 31, 41, 61, 62, 71, 110) can be set in a specific angular range, the rounded outer contour (18) being formed in particular as approximately spherical, spiral, parabolic, elliptical or hyperbolic.

2. Fixing device according to Claim 1, **characterized in that** the fixing device is provided in the clamping region (13) with at least one slit (15), which extends at least partially in the direction of the opening (12) and ends at least partially in the latter.

3. Fixing device according to either of Claims 1 and 2, **characterized in that** the fixing device (10, 10') is formed in the clamping region (13) as a screw head.

4. Fixing device according to Claim 3, **characterized in that** the fixing device (10, 10') is formed as a screw (1) with a screw shank (2) with a screw thread (3) or as a pin with a shank without a screw thread.

5. Fixing device according to either of Claims 3 and 4, **characterized in that** the screw head (2) is provided, in particular above the clamping region (13), with an extension (4) for applying a torque to the fixing device (10, 10').

6. Fixing device according to one of Claims 2 to 5, **characterized in that** the slit (15) is arranged at least in the clamping region (13) and at most along the entire length of the main body (11, 11').

7. Fixing device according to one of Claims 1 to 6, **characterized in that** the opening (12) has an inner surface (17), which is at least partially structured.

8. Combination of a fixing device according to one of Claims 1 to 7 and an elongated element (20, 30, 30', 40, 50, 60, 80, 90, 100, 120, 131, 141), the elongated element being selected from the group comprising wire, rod, pin, profile, spring, hollow tube and scalpel.

9. Combination according to Claim 8, **characterized in that** the elongated element (80, 100) has at least partially a structured surface.

10. Combination according to either of Claims 8 and 9, **characterized in that** the elongated element is provided at least partially with a thread, a drill helix and/or a knurl (80, 100).

11. Combination according to either of Claims 8 and 9, **characterized in that** the elongated element (90, 100) has a tip (91), which is formed as a lancet or a trocar, or which is provided with a self-cutting or self-drilling thread (101).

12. Assembly with a combination according to one of Claims 8 to 11 and with a retaining structure (21, 31, 41, 61, 62, 71, 110) with at least one receptacle (23, 33, 43, 63, 113) for receiving the fixing device (10, 10').

13. Assembly according to Claim 12, **characterized in that** the receptacle and the fixing device are formed in such a way that the elongated element can be secured by the fixing device in a plurality of different angular positions in relation to the retaining structure.

14. Assembly according to Claim 12 or 13, **characterized in that** the at least one receptacle (23, 33, 43, 63, 113) is provided on its inner wall (65) with at least one radially inwardly extending taper (64).

15. Assembly according to one of Claims 12 to 14, **characterized in that** the inner wall (65) is provided, seen in the longitudinal direction (A), with a rounded inner contour, in particular is formed as at least approximately spherical, spiral, parabolic, elliptical or hyperbolic.

16. Assembly according to one of Claims 12 to 15, **characterized in that** the retaining structure is selected from the group comprising
- a retaining structure (51) for receiving an endoscopy tube (50), the elongated element being formed as a tube or a trocar,
- a temporary, intraoperative supporting element (21), in particular a fixing ring for an arthrodesis set,
- a surgical frame that can be fixed on the operating table,
- an internal fixator (41), a wire fixator, a cable fixator or an intramedullary pin,
- an external fixator (31) and
- a scalpel holder, the elongated element being formed as a scalpel blade (120) or as a scalpel shaft (121).

17. Osteosynthesis set, comprising at least two implant plates (61, 62; 71, 72) or one implant plate with at least two portions
which are respectively provided with at least one receptacle (63) for receiving a connecting assembly (10, 60; 70, 73; 10, 73, 100)
and with at least one connecting assembly (10, 60; 70, 73; 10, 73, 100), which can be inserted into a receptacle (63) of each of the implant plates (61, 62; 71, 72) or each of the portions,
wherein the implant plates (61, 62; 71, 72) or the portions thereof can be connected to one another and stabilized by means of the connecting assembly (10, 60; 70, 73; 10, 73, 100) of the implant plates, the connecting assembly having at least one fixing device (10, 10') according to one of Claims 1 to 7 and at least one elongated element, in particular a connecting wire (60, 70, 100).

18. Osteosynthesis set according to Claim 17, **characterized in that** the elongated element (60, 70, 100) has a thread at at least one end and a connecting element, in particular a nut (73), into which the thread can be screwed and by means of which the elongated element (60, 70, 100) can be secured in one of the implant plates (72).

## Revendications

1. Dispositif de fixation (10, 10') pour la fixation d'un élément allongé (20, 30, 30', 40, 50, 60, 80, 90, 100, 120, 131, 141), en particulier d'un fil métallique, d'une goupille ou d'un tube, dans un logement (23, 33, 43, 63, 113) d'une structure de retenue (21, 31, 41, 61, 62, 71, 110), en particulier d'une plaque,
comprenant un corps de base (11, 11') qui présente une ouverture (12, 12'), en particulier un alésage traversant, pour recevoir l'élément allongé,
le corps de base (11, 11') pouvant être déformé radialement par rapport à l'élément allongé au moins dans une région de serrage (13, 13') de telle sorte que la dimension (D) de l'ouverture, en particulier le diamètre de l'alésage (12), puisse être réduit(e),
le corps de base (11, 11') présentant un contour extérieur (14), qui est réalisé de telle sorte que lors de l'insertion du dispositif de fixation (10, 10') dans le logement (23, 33, 43, 63, 113), la région de serrage (13) puisse être déformée de telle sorte que l'élément allongé (20, 30, 30', 40, 50, 60, 80, 90, 100, 120, 131, 141) puisse être serré fixement dans l'ouverture, en particulier l'alésage (12),
le dispositif de fixation (10, 10') étant pourvu dans la région de serrage (13) sur le contour extérieur (14) d'au moins une surface de serrage (16) se rétrécissant radialement au moins partiellement dans la direction périphérique (U), qui, par la rotation du corps de base, peut être amenée en contact avec des saillies clavetées correspondantes d'un logement (23, 33, 43, 63, 113) d'une structure de retenue (21, 31, 41, 61, 62, 71, 110), de sorte qu'il en résulte des forces radiales qui conduisent à une compression radiale du corps de base,
**caractérisé en ce que**
le dispositif de fixation (10, 10') présente un corps de base (11, 11') qui présente, vu au moins partiellement dans la direction longitudinale (A), un contour extérieur arrondi (18), de sorte que l'orientation du dispositif de fixation (10, 10') dans le logement (23, 33, 43, 63, 113) de la structure de retenue (21, 31, 41, 61, 62, 71, 110) puisse être ajustée dans une plage angulaire déterminée, le contour extérieur arrondi (18) étant réalisé en particulier sous forme approximativement sphérique, en spirale, parabolique, elliptique ou hyperbolique.

2. Dispositif de fixation selon la revendication 1, **caractérisé en ce que** le dispositif de fixation est pourvu dans la région de serrage (13) d'au moins une fente (15), qui s'étend au moins en partie dans la direction de l'ouverture (12) et qui se termine au moins en partie dans celle-ci.

3. Dispositif de fixation selon l'une quelconque des revendications 1 à 2, **caractérisé en ce que** le dispositif de fixation (10, 10') est réalisé dans la région de serrage (13) sous forme de tête de vis.

4. Dispositif de fixation selon la revendication 3, **caractérisé en ce que** le dispositif de fixation (10, 10') est réalisé sous forme de vis (1) avec une tige de vis (2) avec un filetage (3) ou sous forme de goupille avec une tige sans filetage.

5. Dispositif de fixation selon l'une quelconque des revendications 3 ou 4, **caractérisé en ce que** la tête de vis (2), en particulier au-dessus de la région de serrage (13), est pourvue d'une saillie (4) pour solliciter le dispositif de fixation (10, 10') avec un couple.

6. Dispositif de fixation selon l'une quelconque des revendications 2 à 5, **caractérisé en ce que** la fente (15) est disposée au moins dans la région de serrage (13) et au maximum le long de toute la longueur du corps de base (11, 11').

7. Dispositif de fixation selon l'une quelconque des revendications 1 à 6, **caractérisé en ce que** l'ouverture (12) présente une surface interne (17) qui est au moins partiellement structurée.

8. Combinaison d'un dispositif de fixation selon l'une quelconque des revendications 1 à 7 et d'un élément allongé (20, 30, 30', 40, 50, 60, 80, 90, 100, 120, 131, 141), l'élément allongé étant sélectionné parmi le groupe constitué d'un fil métallique, d'une barre, d'une goupille, d'un profilé, d'un ressort, d'un tube creux et d'un scalpel.

9. Combinaison selon la revendication 8, **caractérisée en ce que** l'élément allongé (80, 100) présente au moins en partie une surface structurée.

10. Combinaison selon l'une quelconque des revendications 8 ou 9, **caractérisée en ce que** l'élément allongé est pourvu au moins en partie d'un filetage, d'un foret hélicoïdal et/ou d'un moletage (80, 100).

11. Combinaison selon l'une quelconque des revendications 8 ou 9, **caractérisée en ce que** l'élément allongé (90, 100) présente une pointe (91) qui est réalisée sous forme de lancette ou de trocart, ou qui est pourvue d'un filetage (101) auto-taraudeur ou autoperforant.

12. Agencement comprenant une combinaison selon l'une quelconque des revendications 8 à 11, ainsi qu'une structure de retenue (21, 31, 41, 61, 62, 71, 110) comprenant au moins un logement (23, 33, 43, 63, 113) pour recevoir le dispositif de fixation (10, 10') .

13. Agencement selon la revendication 12, **caractérisé en ce que** le logement et le dispositif de fixation sont réalisés de telle sorte que l'élément allongé puisse être fixé avec le dispositif de fixation dans une pluralité de positions angulaires différentes par rapport à la structure de retenue.

14. Agencement selon la revendication 12 ou 13, **caractérisé en ce que** l'au moins un logement (23, 33, 43, 63, 113) est pourvu sur sa paroi interne (65) d'au moins un rétrécissement (64) s'étendant radialement vers l'intérieur.

15. Agencement selon l'une quelconque des revendications 12 à 14, **caractérisé en ce que** la paroi interne (65), vue dans la direction longitudinale (A), est pourvue d'un contour interne arrondi, en particulier est réalisée au moins approximativement sous forme sphérique, en spirale, parabolique, elliptique ou hyperbolique.

16. Agencement selon l'une quelconque des revendications 12 à 15, **caractérisé en ce que** la structure de retenue est sélectionnée parmi le groupe constitué :
- d'une structure de retenue (51) pour recevoir un tube d'endoscopie (50), l'élément allongé étant réalisé sous forme de tube ou de trocart,
- d'un élément de support (21) temporaire peropératoire, en particulier une bague de fixation pour un kit pour arthrodèse,
- d'un cadre opératoire, qui peut être fixé sur la table d'opération,
- d'un fixateur interne (41), d'un fixateur à fil métallique, d'un fixateur à câble ou d'un clou médullaire,
- d'un fixateur externe (31),
- d'une retenue pour scalpel, l'élément allongé étant réalisé sous forme de lame de scalpel (120) ou de tige de scalpel (121).

17. Kit d'ostéosynthèse, constitué d'au moins deux plaques d'implant (61, 62 ; 71, 72) ou d'une plaque d'implant avec au moins deux portions,
qui sont pourvues d'au moins un logement (63) respectif pour recevoir un agencement de connexion (10, 60 ; 70, 73 ; 10, 73, 100)
et avec au moins un agencement de connexion (10, 60 ; 70, 73 ; 10, 73, 100) qui peut être inséré dans un logement respectif (63) des plaques d'implant (61, 62 ; 71, 72) ou des portions,
les plaques d'implant (61, 62 ; 71, 72) ou leurs portions pouvant être connectées et stabilisées les unes aux autres au moyen de l'agencement de connexion (10, 60 ; 70, 73 ; 10, 73, 100) des plaques d'implant,
l'agencement de connexion présentant au moins un dispositif de fixation (10, 10') selon l'une quelconque des revendications 1 à 7 ainsi qu'au moins un élément allongé, en particulier un fil de connexion (60, 70, 100).

18. Kit d'ostéosynthèse selon la revendication 17, **caractérisé en ce que** l'élément allongé (60, 70, 100) présente un filetage sur au moins une extrémité et un élément de connexion, en particulier un écrou (73), dans lequel le filetage peut être vissé, et au moyen duquel l'élément allongé (60, 70, 100) peut être fixé dans l'une des plaques d'implant (72).
